# EUROPEAN PATENT APPLICATION

(11) **EP 3 721 843 A1**
(43) Date of publication of application: **14.10.2020**
(21) Application number: 19168825.8
(22) Date of filing: 12.04.2019
(51) Int. Cl.: A61F 13/42

(54) **ABSORBENT ARTICLE COMPRISING ATTACHMENT REGION**

(71) Applicant: The Procter & Gamble Company, Cincinnati, OH 45202 (US)
(72) Inventor: ARIZTI, Blanca, 65824 Schwalbach am Taunus (DE); KINDSCHUH, Olivia Paige, Cincinnati, OH 45202 (US)
(74) Representative: P&G Patent Germany

(57) **Abstract**

The present invention relates to absorbent articles comprising at least one attachment region for securing a wearable sensing device and further comprising visible placement indicia aligned with the attachment region wherein the visible placement indicia aids the placement of the wearable sensing device on the absorbent article. The present invention further relates to a system comprising the absorbent article and the wearable sensing device.

The present invention further relates to a method for manufacturing an absorbent article comprising visible placement indicia aligned with a wetness indicator and/or attachment region.

## Description

### Field of the Invention

The present invention relates to an absorbent article comprising attachment regions wherein an attachment region is provided for securing a wearable sensing device to the absorbent article.

### Background of the Invention

An absorbent article may be adapted to secure a wearable sensing device to the absorbent article, for example, to the outside (garment facing side) of the absorbent article.

For example an absorbent article may comprise a wetness indicator which is in liquid communication with the absorbent core, and which is visible through the back sheet, from the garment side of the absorbent article. The wetness indicator is typically designed to change colour when urine comes into contact with the wetness indicator. The change in colour of the wetness indicator may be discernable by the human eye, or the colour change may be difficult to distinguish by the human eye alone, or the colour change may even be indistinguishable to the human eye.

A wearable sensing device which is correctly positioned with respect to the wetness indicator can reliably detect changes in colour of the wetness indicator, even to detect small changes in shade, whether or not those colour changes are discernable to the human eye. Information about the state of wetness of the absorbent article can be transmitted from the wearable sensing device to the care-giver, which may inform the care-giver of the need to change the absorbent article.

Typically the absorbent article is a disposable article used for personal hygiene, meaning that the article is disposed of after use and replaced by a fresh article. The wearable sensing device can be reused multiple times by reaffixing it to a fresh absorbent article following a change.

When the wearable sensing device is attached to the absorbent article, the wearable sensing device needs to be securely attached to avoid or minimise the risk of the sensing device being removed unintentionally or the risk of being removed by someone other than the care-giver, for example by the wearer of the wearable sensing device or by a sibling. Minimising the risk of such removal of the wearable sensing device helps to minimise the risk of a hazard of the wearable sensing device being put into the mouth and presenting a choking hazard.

EP 3 213 727, published on September 6^{th} 2017, discloses an absorbent article and a sensor for detecting, for example, the presence of urine in the absorbent core of the article.

There is a need to provide an attachment region on the garment-facing side of an absorbent article, such as a diaper, which is adapted to receive a wearable sensing device, such as a sensor for detecting wetness, movement, environmental conditions etc.

### Summary of the Invention

The present invention relates to an absorbent article comprising: a longitudinal direction extending from the front to the back of the absorbent article and a transverse direction extending from side to side, the absorbent article comprising a front waist region, a back waist region, and a crotch region between the front and back waist regions; the absorbent article comprising a topsheet, a backsheet and an absorbent core between the topsheet and the backsheet, and comprising at least one attachment region for securing a wearable sensing device; the attachment region being located on the side of the backsheet opposite to the absorbent core;
wherein a visible placement indicia circumscribes a first area, F, and the visible placement indicia extends longitudinally from a leading edge, generally towards the waist region of the absorbent article, to a trailing edge, generally towards the crotch region of the absorbent article, wherein the visible placement indicia aids the placement of the wearable sensing device.

In one embodiment the present invention the attachment region comprises a discrete piece of material having second area, M, affixed to the backsheet and wherein the second area, M, completely overlies the first area, F.

In an alternative embodiment the longitudinal axis of the wetness indicator and the longitudinal axis of the visible placement indicia are parallel, and the transverse displacement between the longitudinal axis of the wetness indicator and the longitudinal axis of the visible placement indicia are parallel is less than 3mm.

The present invention further relates to a method for manufacturing an absorbent article, the method comprising the steps of: providing a backsheet film layer comprising visual placement indicia comprising indicia longitudinal centerline and indicia transverse centerline; applying a wetness indicator to the backsheet, wherein the wetness indicator comprises a wetness indicator longitudinal centerline and a wetness indicator transverse centerline; combining the backsheet and a topsheet with an absorbent core therebetween to define an absorbent article; wherein the wetness indicator transverse centerline and the indicia transverse centerline lie within 3mm of one another.

The present invention further relates to method for manufacturing an absorbent article, the method comprising the steps of: providing a backsheet comprising visual placement indicia comprising indicia longitudinal centerline and indicia transverse centerline; affixing a backsheet nonwoven layer to the backsheet film layer; affixing an attachment region to the backsheet nonwoven layer suitable for releasably attaching a wearable sensor device to the absorbent article, wherein the attachment region comprises an attachment region longitudinal centerline and an attachment region transverse centerline; combining the backsheet and a topsheet with an absorbent core therebetween to define the absorbent article; wherein the attachment region transverse centerline and the indicia transverse centerline are within 3mm of one another.

### Brief Description of the Drawings

Figure 1 is a top view of an absorbent article according to an embodiment of the present invention in the form of a diaper with some layers partially removed;
Figure 2 is a transversal cross-section of the embodiment of Figure 1 at the crotch area;
Figure 3 is a back view of the absorbent article of Figure 1 showing the diaper with the backsheet uppermost;
Figures 4a, 4c, 4e and 4g are schematic views of arrangements of an attachment region, a wetness indicator and a visible placement indicia according to embodiments of the present invention.
Figures 4b, 4d and 4f are schematic views of arrangements of an attachment region, a wetness indicator and a visible placement indicia falling outside of the scope of the present invention.

### Detailed Description of the Invention

Absorbent articles used for personal hygiene typically comprise a topsheet, a backsheet and an absorbent core between the topsheet and the backsheet. Such absorbent articles are typically disposable, and are disposed of after use. The topsheet is typically a liquid-permeable sheet to allow liquid, urine etc., to enter the absorbent core; the backsheet is largely or completely liquid-impermeable to prevent leakage from the absorbent article. Exemplary absorbent article forms include diapers comprising fastening tabs or ears and pants comprising elasticized side panels and/or waistbands.

In one embodiment of the present invention the backsheet may be provided with a wetness indicator. For example the wetness indicator may be a coloured strip which changes colour or shade when exposed to urine. In a most preferred embodiment the wetness indicator is a coloured strip which is printed onto the inside (i.e. body side) of the backsheet. In this embodiment the wetness indicator is in liquid communication with the absorbent core and consequently the wetness indicator is exposed to the same environment as that of the core. When an insult of urine is absorbed by the absorbent core then the wetness indicator responds. In preferred embodiments the wetness indicator responds to the presence of urine by changing colour or shade.

In a preferred embodiment the wetness indicator is a coloured strip, for example a strip of about 10 mm width, lying longitudinally along the centre line of the absorbent article. In one particularly preferred embodiment the wetness indicator comprises a pH sensitive chemical which changes colour or shade when urine (low pH) is present.

A wearable sensing device maybe reusable, and intended to be reused multiple times after multiple changes of disposale absorbent articles.

A wearable sensing device may contain one or more sensors, for example: optical wetness sensor(s); colour wetness sensor(s); chemical sensor(s), including volatile organic chemical (VOC) sensor(s); accelerometer(s); temperature sensor(s); humidity sensor(s); microphone(s); gyroscope(s); magnetometer(s); global positioning sensor(s); and any of these types of sensor in combination.

In embodiments of the present invention the absorbent article comprises an attachment region.

The attachment region preferably comprises mechanically interoperating elements such as outwardly facing fibres. Outwardly facing fibres in the form of "hooks" or "loops" in an attachment region interconnect with corresponding "hooks" or "loops" projecting from the cooperating attachment region. For example "hooks" or "loops" on the external housing of the wearable sensing device such as to enable the wearable sensing device to be attached to the cooperating side of the garment-facing side of the absorbent article and subsequently detached. Most preferably the garment-facing side of the absorbent article is provided with an attachment region that comprises interconnecting "hooks" or "loops". By way of example only, fiber loops assocated with an attachment region may arise from an outer cover nonwoven layers that forms all or a majority the outermost layer of the backsheet of the absorent article. Alternatively loops associated with an attachment region may arise from a discrete peice of material that is adhered to the absorbent article backsheet even where the outermost layer of the backsheet comprise a fibrous material.

The wearable sensing device is thus attached to the outside (i.e. garment side) of the backsheet. In an embodiment comprising a wetness indicator, the wearable sensing device should be in sufficiently close proximity to the wetness indicator so that the wearable sensing device senses changes in colour of the wetness indicator. The changes of colour which may be sensed by the wearable sensing device may be imperceptible, or barely perceptible, to the human eye, or the changes of colour may also be visible to the human eye. Light should be transmitted through the backsheet and through the attachment region sufficient to enable the wearable sensing device to sense changes, even small changes, in colour of the wetness indicator. Preferably the opacity of the combination of the backsheet and the atachment region should be less than 80%, more preferably less than 60%.

The wetness indicator is visible from the outside (garment side) of the absorbent article. The outside (garment side) of the absorbent article also comprises visible placement indicia to indicate the optimum position for the placement of the wearable sensing device and to aid the placement of the wearable sensing device.

The wetness indicator is a strip having width, W, wherein the strip extends in the longitudinal direction. The visible placement indicia to aid the placement of the wearable sensing device circumscribes a first area, F, and the visible placement indicia extends longitudinally from a leading edge, generally towards the waist region of the absorbent article, to a trailing edge, generally towards the crotch region of the absorbent article.

A second area, M, is covered by the discrete material of the attachment region that is affixed to the backsheet. The discrete material of the attachment region extends longitudinally from a leading edge, generally towards the waist region of the absorbent article, to a trailing edge, generally towards the crotch region of the absorbent article.

In a preferred embodiment the second area, M, completely overlies the first area, F.

In another preferred embodiment the wetness indicator extends from the leading edge of the first area, F, to the trailing edge of the first area, F. In a still more preferred embodiment the wetness indicator extends from the leading edge of the discrete material of the attachment region to the trailing edge of the discrete material of the attachment region.

Values for peel force and shear force are preferably determined using ASTM D5169-98 for shear strength (dynamic method) of hook and loop touch fasteners; and ASTM D5170-98 for peel strength ("T" method) of hook and loop touch fasteners. Other test methods are also described herein.

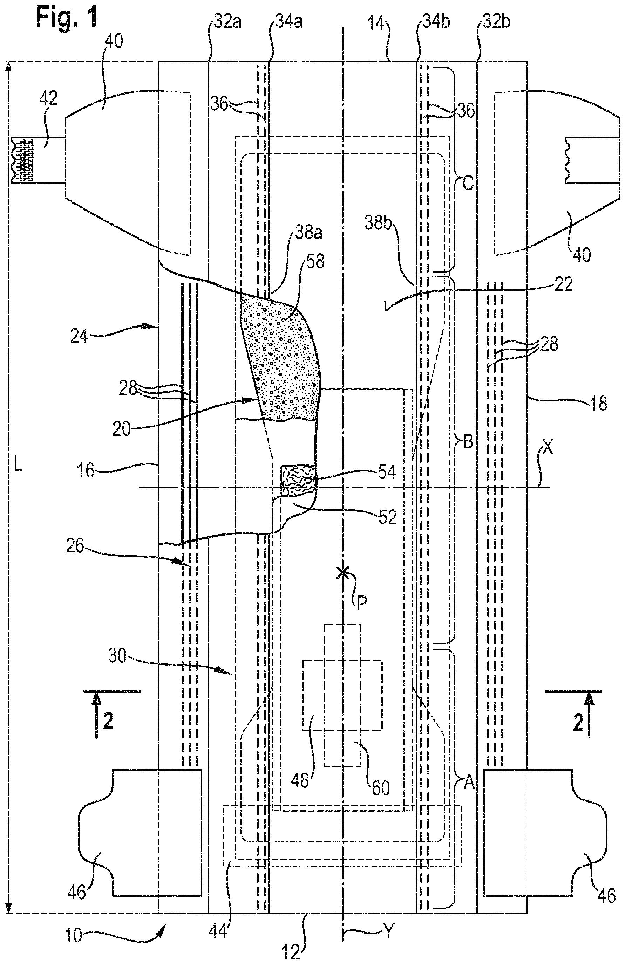

An exemplary absorbent article according to the invention in the form of a diaper 10 is represented in Figs. 1, 2 and 3. However it is to be understood that the present invention is not limited to a taped diaper as illustrated. The present invention may equally apply to other forms of absorbent articles such as pants, training pants, bedwetter pants, adult incontinence pads or pants etc. which may comprise an elastic belt. In such pants an absorbent core / chassis assembly is typically provided in conjunction with the elastic belt. The arrangement of wetness indicator, visible placement indicia and attachment region may be provided on the backsheet at a position opposite the absorbent core.

Fig. 1 is a plan view of the exemplary diaper 10, in a flattened state, with portions of the structure being cut-away to more clearly show the construction of the diaper 10. This diaper 10 is shown for illustration purpose only as the invention may be used for making a wide variety of diapers or other absorbent articles. The diaper extends from a front edge 12 to a longitudinally opposed rear edge 14. It comprises left side edge 16 and transversally opposed right side edge 18. The diaper 10 comprises an absorbent core 20 which is positioned between topsheet 22, which is at least partially liquid permeable and backsheet 24, which is essentially impermeable to liquid.

In Figure 1 "X" denotes a transversal access through the geometrical center of the diaper, and axis "Y" denotes the longitudinal direction. The area A denotes the front area of the diaper as seen in the longitudinal direction and C denotes the rear area of the diaper as seen in the longitudinal direction, and B denotes the central area or crotch area positioned between area A and area B, in the longitudinal direction. L denotes the length of the diaper from the front edge 12 to rear edge 14 as measured in the longitudinal direction.

The article comprises a crotch point P defined herein as the point placed on the longitudinal axis at a distance of two fifth (2/5) of L starting from the front edge 12 of the diaper 10.

The absorbent article may comprise a wetness indicator 60, which can be printed on to the backsheet, preferably printed on the surface of the backsheet adjacent to the absorbent core. Alternatively the wetness indicator 60 may take the form of a small sheet of material or patch placed in liquid communication with the absorbent core 20. Still further, the wetness indicator may be positioned on a surface of a core material or core nonwoven adjacent to the backsheet film. As shown, a rectangular form is useful. The wetness indicator 60 can be arranged in the front area A, the central area B, the rear area C of the diaper, or combinations thereof. It is often useful to range the wetness indicator 60 in the central area B or in the front area A. As shown, it can be useful to provide the wetness indicator 60 towards the front of the crotch point P.

The diaper 10 further comprises gasketing cuffs 26 for maintaining a tight fit of the diaper 10 to the wearer, when the diaper 10 is worn. The gasketing cuffs 26 comprise elastics 28 for maintaining the tight fit, which helps to avoid leakage.

The diaper 10 further comprises barrier leg cuffs 30 on each side of the diaper. Barrier leg cuffs comprise proximal edges 32a and 32b, which are adjacent to topsheet 22. Opposed to the respective proximal edges, the barrier leg cuffs 30 comprise distal edges 34a and 34b, respectively. In the area of the distal edges 34, further elastics 36a provided, while a portion of the distal edges 34 of the barrier leg cuffs 30 can be attached to components of the diaper 10, such as the topsheet 22, it is preferred that the barrier leg cuffs 30 also comprise unattached areas of the distal edges, herein referred to as free flaps 38. The respective free flaps 38 are typically provided in the central region of the diaper 10.

The diaper 10 further comprises the fastening system, for fastening the diaper to the body of a wearer. This fastening system comprises two back ears 40, which comprise adhesive tapes 42. The adhesive tapes 42 can be attached to landing zone region 44. In the front area, the diaper comprises front ears 46. As described below, for other embodiments other fastening systems can be useful, including mechanical fasteners.

The diaper 10 may further comprise attachment region 48 for securing a wearable sensing device to the diaper. Alternatively, the diaper may comprise an outer cover nonwoven on its garment facing side and adjacent to a backsheet film or a film/nonwoven laminate wherein the garment facing nonwoven surface is for securing a wearable sensing device to the diaper.

The core can optionally comprise areas, where there is a reduced amount of absorbent material or no absorbent material. These areas are referred to as channels.

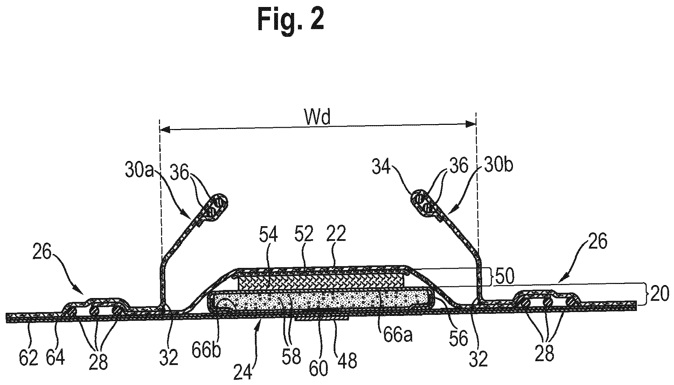

Figure 2 is transversal cross-section of the embodiment of Figure 1 and readily shows other structural elements of the diaper. As shown in this figure, the diaper comprises an acquisition-distribution system 50. This acquisition-distribution system comprises acquisition layer 52, which first receives liquid, and distribution layer 54 underneath acquisition layer 52.

The absorbent core 20 comprises a core layer 56. This core layer can comprise particular material 58, such as super absorbent particles, herein also referred to SAP. Between the core layer 56 and the backsheet 24 the wetness indicator 60 can be arranged. As shown in Fig. 2 the backsheet 24 can comprise and inner backsheet layer 62 (which is oriented towards the core 20) and an outer backsheet layer 64, which is generally oriented towards the garments of a wearer. As shown in Fig. 2, in accordance with the present invention, the wetness indicator 60 can be provided above the inner backsheet layer and below the core wrap 66, more precisely, below the portion of the core wrap 66 which is oriented towards the backsheet 24. The attachment region 48 is provided on the outer backsheet layer 64. Preferably the attachment region 48 is a discrete material from the backsheet 24 and the discrete attachment region 48 is attached, for example adhesively attached, to the backsheet 24. A wearable sensing device (not shown) can be releasably affixed to the attachment region 48 so that the wearable sensing device is correctly positioned with respect to the wetness indicator 60.

In one embodiment of the present invention the discrete attachment region 48 is affixed to the backsheet 24 during the assembly of the absorbent article. In this embodiment the attachment region 48 is attached in-line, i.e. by a processing step carried out as one step of the assembly of the absorbent article on a converting apparatus. For example, the attachment region 48 may be attached by a conventional cut and slip process. In an alternative embodiment of the present invention the discrete attachment region 48 is attached to the backsheet 24 off-line, before the backsheet 24 is assembled together with the rest of the absorbent article. For example, the attachment region 48 may be combined with inner and outer backsheet layers to form a trilaminate. The trilaminate, including the attachment layer, may be stored, for example on a roll of material, and subsequently transported to the converting apparatus or festooned. The trilaminate is then assembled together with the rest of the absorbent article.

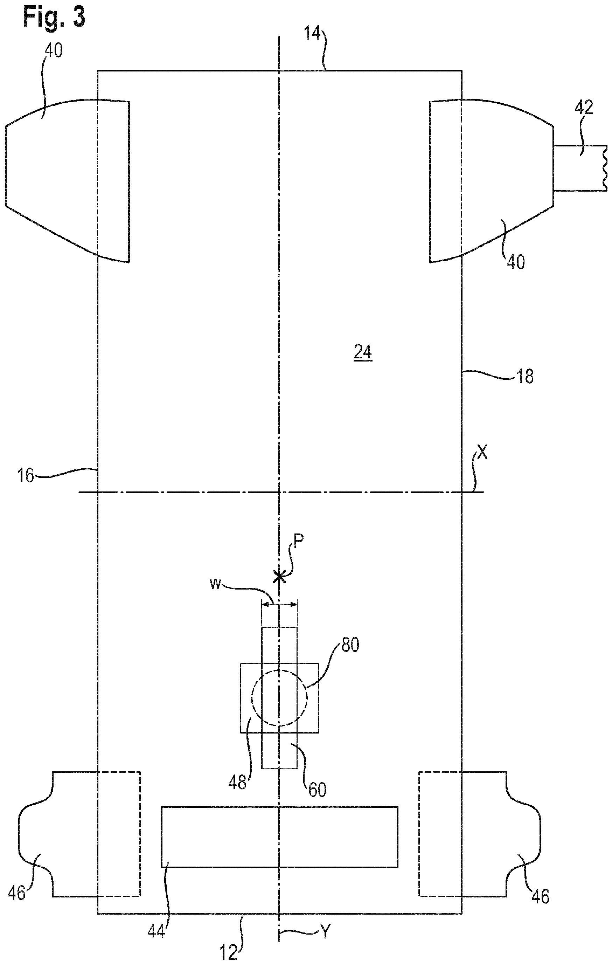

Fig. 3 is a plan view of the exemplary diaper 10, in a flattened state, showing the reverse side to that depicted in Fig. 1. The diaper in Fig. 3 is shown with the backsheet 24 uppermost, and also shows a wetness indicator 60, a visible placement indicia 80 and an attachment region 48. Attachment region 48 may comprise a portion of an overall material layer that forms the outermost layer of exemplary diaper 10, or may alternatively comprise a discrete piece of material that is affixed to backsheet 24. The wetness indicator 60 is in the form of a strip having width, W, lying on the longitudinal axis, Y, of the diaper. The visible placement indicia 80 circumscribes first area, F, and extends longitudinally from a leading edge which lies generally towards the waist region to a training edge which lies generally towards the crotch region. The visible placement indicia 80 aids the placement of the wearable sensing device.

The wetness indicator 60, for example, may be disposed in the diaper such as shown in Fig. 3 generally along a mid-line of the diaper and extend a predetermined width, W, in the transverse direction of the diaper. In one particular embodiment, for example, the wetness indicator is between approximately 4 mm and 15 mm wide, such as approximately 10 mm wide, and between approximately 100 and 300 mm long, such as approximately 100 mm, 150 mm, 200 mm, 250 mm or 300 mm long. The dimensions of the color change strip, however, are merely exemplary and not limiting.

The visible placement indicia 80 comprises an outline which generally corresponds to the outer profile of the wearable sensing device. In Fig. 3 the visible placement indicia 80 is "egg" shaped, but may equally be any other shape including circular, elliptical, square, rectangular etc. By way of example the visible placement indicia shown in Fig. 3 is about 55 mm long and about 40 mm at the widest part. The visible placement indicia 80 may be printed, for example printed onto either the front or the back of the backsheet 24, or onto either side of a component sheet 62, 64 in case the backsheet is a laminate, or the visible placement indicia 80 could be enabled by some structural manipulation of material, for example aperturing, needling, embossing, etc. of at least one layer of the backsheet 24.

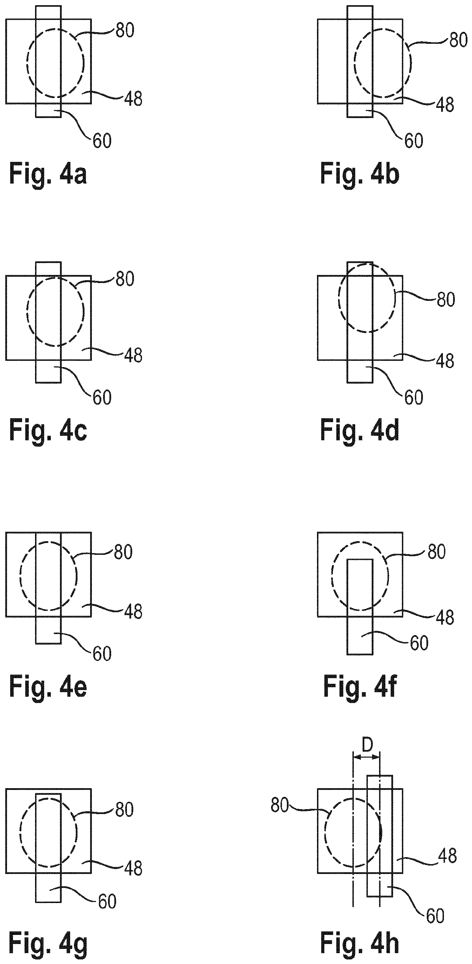

Figures 4a to 4g show various different arrangements of wetness indicator 60, visible placement indicia 80 and attachment region 48.

Figure 4a is a schematic view showing the discrete piece of the attachment region 48 having second area, M, completely overlies the first area, F, circumscribed by the visible placement indicia. This is an embodiment of the present invention.

Figure 4b is a schematic view showing the first area, F, circumscribed by the visible placement indicia 80 extending beyond the transverse edge of the discrete piece of the attachment region 48. This embodiment falls outside of the present invention.

Figure 4c is a schematic view showing the discrete piece of the attachment region 48 having second area, M, completely overlies the first area, F, circumscribed by the visible placement indicia 80. This is an embodiment of the present invention.

Figure 4d is a schematic view showing the first area, F, circumscribed by the visible placement indicia 80 extending beyond the longitudinal edge of the discrete piece of the attachment region 48. This embodiment falls outside of the present invention.

Figure 4e is a schematic view showing a wetness indicator 60 extending from the leading edge of the attachment region having second area, M, to beyond the trailing edge of the attachment region 48. This is an embodiment of the present invention.

Figure 4f is a schematic view showing wetness indicator 60 extending only across part of the second area, M, of the attachment region 48 and indeed only in part across the first area, F, circumscribed by the visible placement indicia 80. This embodiment falls outside of the present invention.

Figure 4g is a schematic view showing wetness indicator 60 extending at least across the leading edge of the first area, F, at least to the trailing edge of the first area, F. This is an embodiment of the invention.

Figure 4h is a schematic view showing that the longitudinal axis of the wetness indicator, 60, and the longitudinal axis of the visible placement indicia are transversely displaced by a distance, D. This is an embodiment of the invention provided D < 3mm, preferably D < 2mm, and more preferably D < 1 mm. Most preferably there is no transverse displacement between the two axes, and D=0.

### Wearable Sensing Device

The wearable sensing device comprises a housing which contains various components, such as electronic components. The housing maybe a rigid housing or it maybe a flexible or partly flexible housing. The components contained by the housing may be selected from the list comprising: data processing unit, memory storage, power source, light emitting device, sensors, data transmission unit, antenna, output units such as indicators, display unit and some or all of the units in combination.

In one embodiment of the invention the sensor, or one of the sensors, is a color sensor which can generate an output which depends on a color observed by the color sensor. It can also be referred to as an optical sensor. A useful color sensor can comprise a photo-diode. For example color sensor TCS 34725, commercially available from AMS-TAOS USA Inc., Plano, Texas, has been found useful. The TCS 34725 device provides a digital return of red, green, blue (RGB). An IR blocking filter, integrated on-chip and localized to the color sensing photodiodes, minimizes the IR spectral component of the incoming light and allows color measurements to be made accurately.

The wearable sensing device may also comprise a light emitting device, such as an LED, for emitting light onto an area, the color of which is to be assessed by the color sensor. The color sensor is in particular optimized for assessing the color of the color-changing indicator. The color sensor might be sensitive to visible and non-visible light, namely light in the near IR range. Sensor of this type are referred to as hyperspectral sensors. As discussed above the wetness indicator can change its color, and preferably does change its color, based on the presence of bodily exudates. The color sensor, when positioned appropriately with respect to the wetness indicator, will therefore provide an output, which varies depending on the presence of bodily exudates.

While the description and the figures focus on a sensor that reads a wetness indicator, optical or color sensors that sense coloration change in the absorbent materials associated with the absorbent core are also contemplated, wherein a wetness indicator is unnecessary and therefore eliminated from the absorbent article. In these embodiments, the absorbent article will comprise an attachment region and a visible placement indicia for the wearable sensing device, without the inclusion of a separate wetness indicator. Similarly, other wetness sensor configurations may be employed that do not rely on optical properties of the article and/or wetness indicator associated with the same. For example, capacitive or inductive type wetness sensors can be used in the present invention. Sensors for detecting a bowel movement may also be employed in the wearable sensing device and can comprise optical sensors or other sensors like volatile organic compound sensors.

The wearable sensing device may also comprise motion sensors. Suitable motion sensors may be selected from accelerometer(s), gyroscope(s), magnetometer(s), or combinations of these sensors.

The wearable sensing device may also comprise a data transmission unit which transmits data, preferably wirelessly. The skilled person is aware of useful standards for providing such data transmission, for example Bluetooth, BTLE, mesh (e.g., IEEE 802.15.4), WiFi (e.g., IEEE 802.15.11), communication incorporating all or any portion of IEEE 802 or similar communication standards, RFID, 3G, 4G, 5G communication, Backscatter communication, light communication, audio/sound communication, harvesting protocol communication (e.g., a metadata harvesting protocol). Other communications protocols or combinations of communications protocols (e.g., a Bluetooth/Mesh combined protocol) can be employed. Additionally or alternatively an acoustic or optical broadcasting is useful.

The wearable sensing device may comprise a power source which is designed to provide sufficient electrical power to operate the device for a period of time, for example up to three months, or up to six months. Alternatively, the wearable sensing device may comprise a rechargeable power source.

The wearable sensing device may contain all of the components necessary to operate as intended such that the absorbent article to which it is attached is a typical absorbent article configuration. Alternatively, the wearble sensing device may contain some electical components and the absorbent article to which it is attached contain other electrical components within its interior that work together with those in the wearable sensing device. For example, absorbent articles may comprise electrodes that are wettable with urine and connectable to a wearable sensing device affixed to the outer porition fo the article. In such a configuraiton, the wearable sensing device may comprise a battery, transmitter, and processor that sends a signal to a caregiver upon reading an electrode output.

### Methods of Manufacture

The present invention further relates to a method for manufacturing an absorbent article, the method comprising the steps of: providing a backsheet film layer comprising visual placement indicia comprising indicia longitudinal centerline and indicia transverse centerline; applying a wetness indicator to the backsheet, wherein the wetness indicator comprises a wetness indicator longitudinal centerline and a wetness indicator transverse centerline; combining the backsheet and a topsheet with an absorbent core therebetween to define an absorbent article; wherein the wetness indicator transverse centerline and the indicia transverse centerline lie within 3mm of one another.

Preferably the visual placement indicia and the wetness indicator are disposed on opposite sides of the backsheet. Preferably the wetness indicator longitudinal centerline and the indicia longitudinal centerline lie within 5 mm of one another. Preferably the method further comprises the step of affixing a backsheet nonwoven layer to the backsheet, wherein the backsheet nonwoven layer defines the outermost garment-facing layer of the absorbent article, more preferably the method further comprises the step of affixing an attachment region to the backsheet nonwoven layer suitable for releasably attaching a wearable sensor device to the absorbent article, and still more preferably the attachment region comprises an attachment region longitudinal centerline and an attachment region transverse centerline, and wherein the attachment region transverse centerline, the wetness indicator transverse centerline, and the indicia transverse centerline are all within 3 mm of one another.

The present invention further relates to method for manufacturing an absorbent article, the method comprising the steps of: providing a backsheet comprising visual placement indicia comprising indicia longitudinal centerline and indicia transverse centerline; affixing a backsheet nonwoven layer to the backsheet film layer; affixing an attachment region to the backsheet nonwoven layer suitable for releasably attaching a wearable sensor device to the absorbent article, wherein the attachment region comprises an attachment region longitudinal centerline and an attachment region transverse centerline; combining the backsheet and a topsheet with an absorbent core therebetween to define the absorbent article; wherein the attachment region transverse centerline and the indicia transverse centerline are within 3mm of one another.

Preferably the the attachment region longitudinal centerline and the indicium longitudinal centerline are within 5 mm of one another.

### Package Of Absorbent Articles

A consumer of absorbent articles designed to use with a reusable, wearing sensing device expects all diapers wihtin the purchased package will exhibit a functional, inventive, spatial relationship between a wetness indicator, a visible placement indicia, and an attachment region. A manufacturer of absorbent articles must have a reliable process to yield a series of diaper where a consumer package of these articles will provide the consumer purchasing the package with consistent performance during the use of a wearable sensing device. Hence, on embodiment of the present invention is a package of absorbent articles where all articles contained within the package exhibit a functional, inventive, spatial relationship between a wetness indicator, a visible placement indicia, and an attachment region as exemplified by the various absorbent articles of the present invention.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. An absorbent article comprising: a longitudinal direction extending from the front to the back of the absorbent article and a transverse direction extending from side to side, the absorbent article comprising a front waist region, a back waist region, and a crotch region between the front and back waist regions; the absorbent article comprising a topsheet, a backsheet and an absorbent core between the topsheet and the backsheet, and comprising at least one attachment region for securing a wearable sensing device; the attachment region being located on the side of the backsheet opposite to the absorbent core;
wherein a visible placement indicia circumscribes a first area, F, and the visible placement indicia extends longitudinally from a leading edge, generally towards the waist region of the absorbent article, to a trailing edge, generally towards the crotch region of the absorbent article, wherein the visible placement indicia aids the placement of the wearable sensing device, and wherein the attachment region comprises a discrete piece of material having second area, M, affixed to the backsheet and wherein the second area, M, completely overlies the first area, F.

2. The absorbent article of claim 1 further comprising a wetness indicator in liquid communication with the absorbent core, wherein the wetness indicator is visible when viewed through the backsheet.

3. The absorbent article of claim 2 wherein the wetness indicator extends at least from the leading edge of the discrete material of the attachment region to the trailing edge of the discrete material of the attachment region.

4. The absorbent article of either of claims 1 or 2 wherein wherein the longitudinal axis of the wetness indicator and the longitudinal axis of the visible placement indicia are parallel, and the transverse displacement between the longitudinal axis of the wetness indicator and the longitudinal axis of the visible placement indicia is less than 3mm.

5. The absorbent article of any of claims 2 to 4 wherein the wetness indicator comprises a colour change indicator.

6. An absorbent article comprising: a longitudinal direction extending from the front to the back of the absorbent article and a transverse direction extending from side to side, the absorbent article comprising a front waist region, a back waist region, and a crotch region between the front and back waist regions; the absorbent article comprising a topsheet, a backsheet and an absorbent core between the topsheet and the backsheet, and comprising at least one attachment region for securing a wearable sensing device; the attachment region being located on the side of the backsheet opposite to the absorbent core, and further comprising a wetness indicator in liquid communication with the absorbent core, wherein the wetness indicator is visible when viewed through the backsheet,;
wherein a visible placement indicia circumscribes a first area, F, and the visible placement indicia extends longitudinally from a leading edge, generally towards the waist region of the absorbent article, to a trailing edge, generally towards the crotch region of the absorbent article, wherein the visible placement indicia aids the placement of the wearable sensing device, and wherein the longitudinal axis of the wetness indicator and the longitudinal axis of the visible placement indicia are parallel, and the transverse displacement between the longitudinal axis of the wetness indicator and the longitudinal axis of the visible placement indicia is less than 3mm.

7. The absorbent article of claim 6 wherein the wetness indicator extends at least from the leading edge of the discrete material of the attachment region to the trailing edge of the discrete material of the attachment region.

8. The absorbent article of either of claims 6 or 7 wherein the wetness indicator is disposed on a first side of the backsheet and the visible placement indicia is disposed on opposing second side of the backsheet.

9. The absorbent article of either of claims 6 or 7 wherein the backsheet is a laminate comprising inner backsheet layer and outer backsheet layer, wherein the wetness indicator is disposed on the inner backsheet layer, and the visible placement indicia is disposed on the outer backsheet layer.

10. The absorbent article of either of claims 6 or 7 wherein wherein the backsheet is a laminate comprising inner backsheet layer and outer backsheet layer, and wherein the outer backsheet layer provides the attachment region.

11. The absorbent article of claim 6 wherein the wetness indicator comprises a colour change indicator.

12. A system comprising an absorbent article according to any of the previous claims and further comprising a wearable sensing device, wherein the wearable sensing device is removably attached to the attachment region of the absorbent article, wherein the wearable sensing device comprises one or more sensors selected from the group consisting of: optical wetness sensor; colour wetness sensor, chemical sensor including volatile organic chemical (VOC) sensor; accelerometer; temperature sensor; humidity sensor; microphone; gyroscope; magnetometer; global positioning sensor; and combinations thereof.

13. A method for manufacturing an absorbent article, the method comprising the steps of:
a. providing a backsheet film layer comprising visual placement indicia comprising indicia longitudinal centerline and indicia transverse centerline;
b. applying a wetness indicator to the backsheet, wherein the wetness indicator comprises a wetness indicator longitudinal centerline and a wetness indicator transverse centerline;
c. combining the backsheet and a topsheet with an absorbent core therebetween to define an absorbent article;
d. wherein the wetness indicator transverse centerline and the indicia transverse centerline lie within 3mm of one another.

14. The method of claim 13, wherein the visual placement indicia and the wetness indicator are disposed on opposite sides of the backsheet.

15. The method of claim 13, wherein the wetness indicator longitudinal centerline and the indicia longitudinal centerline lie within 5 mm of one another.

16. The method of claim 13, further comprising the step of affixing a backsheet nonwoven layer to the backsheet, wherein the backsheet nonwoven layer defines the outermost garment-facing layer of the absorbent article.

17. The method of claim 16, further comprising the step of affixing an attachment region to the backsheet nonwoven layer suitable for releasably attaching a wearable sensor device to the absorbent article.

18. The method of claim 17, further comprising the attachment region comprises an attachment region longitudinal centerline and an attachment region transverse centerline, and wherein the attachment region transverse centerline, the wetness indicator transverse centerline, and the indicia transverse centerline are all within 3 mm of one another.

19. A method for manufacturing an absorbent article, the method comprising the steps of:
a. providing a backsheet comprising visual placement indicia comprising indicia longitudinal centerline and indicia transverse centerline;
b. affixing a backsheet nonwoven layer to the backsheet film layer;
c. affixing an attachment region to the backsheet nonwoven layer suitable for releasably attaching a wearable sensor device to the absorbent article, wherein the attachment region comprises an attachment region longitudinal centerline and an attachment region transverse centerline;
d. combining the backsheet and a topsheet with an absorbent core therebetween to define the absorbent article;
e. wherein the attachment region transverse centerline and the indicia transverse centerline are within 3mm of one another.

20. The method of claim 19, wherein the attachment region longitudinal centerline and the indicium longitudinal centerline are within 5 mm of one another.
